(19) Europäisches Patentamt
European Patent Office
Office européen des brevets

(11) EP 2 274 263 B1

(12) **FASCICULE DE BREVET EUROPEEN**

(45) Date de publication et mention de la délivrance du brevet:
**27.09.2017 Bulletin 2017/39**

(21) Numéro de dépôt: **09735913.7**

(22) Date de dépôt: **22.04.2009**

(51) Int Cl.:
***C07C 45/73*** (2006.01) ***B01D 61/36*** (2006.01)
***C07C 45/78*** (2006.01)

(86) Numéro de dépôt international:
**PCT/EP2009/054825**

(87) Numéro de publication internationale:
**WO 2009/130245 (29.10.2009 Gazette 2009/44)**

(54) **PROCÉDÉ D'OPTIMISATION DE PRODUCTION DE CÉTONES OU ALDEHYDES**

VERFAHREN ZUR OPTIMIERTEN HERSTELLUNG VON KETONEN ODER ALDEHYDEN

PROCESS FOR OPTIMIZING THE PRODUCTION OF KETONES OR ALDEHYDES

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **23.04.2008 FR 0802271**

(43) Date de publication de la demande:
**19.01.2011 Bulletin 2011/03**

(73) Titulaire: **Rhodia Poliamida E Especialidades Ltda**
**Sao Paulo - SP (BR)**

(72) Inventeurs:
- **Nasser, Roberto**
**04019-120 Sao Paulo (BR)**
- **Rita, Dalva Janine**
**13023-190 Campinas (BR)**
- **Schwartz, Joël**
**F-69300 Caluire (FR)**

(74) Mandataire: **Ridray, Annabelle et al**
**Rhodia Opérations**
**85, avenue des Frères Perret - BP 62**
**69192 Saint-Fons Cedex (FR)**

(56) Documents cités:
**US-A- 4 910 344 US-B2- 6 960 694**

- **STAUDT-BICKEL C ET AL: "Integration of pervaporation for the removal of water in the production process of methylisobutylketone" JOURNAL OF MEMBRANE SCIENCE, ELSEVIER SCIENTIFIC PUBL.COMPANY. AMSTERDAM, NL, vol. 111, no. 1, 6 mars 1996 (1996-03-06), pages 135-141, XP004041721 ISSN: 0376-7388**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

Le procédé selon l'invention vise à l'optimisation de la production de cétones ou d'aldehydes obtenus par la condensation suivie de la déshydratation et hydrogénation des cétones ou aldehydes dans un réacteur (3), en effectuant une étape de pervaporation membranaire (6) permettant de déshydrater partiellement une fraction sortante du réacteur (5) et de recycler le concentré déshydraté (7) dans ledit réacteur (3).

L'article « Intégration of pervaporation for the removal of water in the production process of methylisobutylketone », Journal of Membrane Science, 1996, 111, 135-141, décrit la production de méthyl isobutyl cétone par la condensation d'acétone suivie de la déshydratation de l'aldol et hydrogénation de la cétone insaturée résultant de la déshydratation.

De façon schématique, le mécanisme de réaction d'obtention de cétones ou aldéhyde de masse supérieure peut être représenté par des réactions élémentaires ; par exemple pour ce qui concerne la méthyl isobutyl cétone (MIBK) :

$$2Acétone \xleftrightarrow{k1} DAA$$

$$DAA \xleftrightarrow{k2} OM + H_2O$$

$$OM + H_2 \xrightarrow{k3} MIBK$$

(DAA= diacétone alcool / OM= oxyde mésityle)
ou alors par la réaction globale :

$$2Acétone + H_2 \xrightarrow{k} MIBK + H_2O$$

Ce qu'il faut retenir des mécanismes présentés, c'est que les réactions impliquées constituent des pseudo-équilibres, dans lesquels le taux de conversion de l'acétone dépend de la concentration d'eau dans la masse réactionnelle, comme indiqué ci-dessous:

$$\frac{dm_{MIBK}}{d\theta} = \frac{W}{V} \cdot K \cdot P_{PH_2} \cdot \frac{x_{acétone}^2}{x_{H_2O}}$$

où :

$\frac{dm_{MIBK}}{d\theta}$ -représente le taux de formation de MIBK;

$\frac{W}{V}$ -représente la masse de catalyseur ;

K - constante caractéristique du system ;

$P_{PH_2}$ - représente la pression partiel de l'hydrogène ;

$x_{acétone}$ - représente la fraction molaire d'acétone ;

$x_{H2O}$ - représente la fraction molaire d'eau.

Autrement dit, plus la teneur en eau de la masse réactionnelle est faible, plus la conversion est forte et vice-versa.

Le réacteur opère à une température entre 50 et 150°C et à une pression entre 500 et 2000 kPa, et la concentration d'eau à la sortie du réacteur est de l'ordre de 3 à 5 % en poids.

La sortie du réacteur alimente classiquement un ensemble de colonnes, dans lesquelles la MIBK est purifiée, la séparant de l'acétone, qui retourne à la réaction.

La présente invention a pour objet de développer un nouveau procédé de production de cétones ou aldéhydes, par exemple la MIBK en réduisant la concentration d'eau dans le milieu réactionnel d'environ 1% en poids, afin d'obtenir un déplacement de l'équilibre vers le côté du produit et ainsi un rendement accru de la réaction, une augmentation de la productivité, et une économie d'énergie.

De telles propriétés ont put etre obtenues par l'utilisation dans un procédé d'obtention de cétones ou aldehydes obtenus par la condensation suivie de la déshydratation et hydrogénation de cétones ou aldehydes dans un réacteur, d'une boucle permettant la récupération d'une fraction sortante du réacteur, comprenant par exemple un mélange d'eau,

d'acétone et de MIBK dans la cas de la fabrication du MIBK, pour l'acheminer vers un module de pervaporation membranaire de façon à la déshydrater partiellement puis la renvoyer dans ledit réacteur.

En effet, il apparait de manière tout à fait surprenante qu'une déshydration même partielle du milieu réactionnel permettait d'obtenir un rendement accru de la réaction et une forte augmentation de la productivité de cétones ou aldehydes.

La déshydratation partielle de la masse réactionnelle d'approximativement 1% en poids déplace l'équilibre de toute réaction de pseudo-équilibre vers le côté de la formation du produit; ceci augmente le rendement de la réaction et donc la productivité du système, et, en conséquence, résulte en une économie d'énergie, ce qui ne se limite pas uniquement à la production de MIBK, mais peut être étendu à d'autres produits obtenus par des réactions de pseudo-équilibre semblables, comme, par exemple, le butanal, obtenu à partir de l'acétaldéhyde.

Les procédés de pervaporation membranaires sont des procédés de séparation extrêmement efficaces, généralement sans changement de phase et permettant ainsi une réduction significative de la consommation d'energie, qui utilisent des membranes sélectives, dans lesquels le flux d'alimentation est tangentiel à la membrane, ou aux membranes.

Il a été démontré par ailleurs qu'un tel procédé permet d'obtenir de meilleurs performances que les traitements en fin de ligne, où l'on cherche classiquement à utiliser les procédés à membranes, comme, par exemple le traitement d'effluents utilisant l'osmose inverse pour réutiliser l'eau et la déshydratation de solvants par pervaporation, soit pour optimiser ou compléter la distillation, soit pour réduire la consommation d'énergie soit pour obtenir le solvant pur. Par ailleurs, le système selon l'invention est plus compact que les systèmes traditionnels de séparation, et possède une plus grande flexibilité opérationnelle au niveau industriel, du fait des structures modulaires, et offre également des coûts d'entretien moindres puisque les systèmes à membranes ne comportent pas de parties mobiles.

La présente invention a ainsi pour premier objet un procédé de production de cétones ou aldehydes obtenus par la condensation suivie de la déshydratation et hydrogénation de cétones ou aldehydes dans un réacteur, caractérisé en ce que ledit procédé comprend au moins une étape d'élimination partielle d'eau effectuée par au moins un module de pervaporation membranaire, alimenté tangentiellement et installé latéralement audit réacteur et fonctionnant en boucle, de façon à déshydrater partiellement une fraction sortante du réacteur et à recycler le concentré déshydraté dans ledit réacteur, de sorte que la concentration d'eau dans la masse réactionnelle du réacteur soit maintenue entre 2 et 4 % en poids, et en ce que la cétone est la méthyl isobutyl cétone ou MIBK, obtenu à partir de l'hydrogénation d'acétone, ou le butanal obtenu à partir d'acétaldéhyde. D'une manière préférentielle la réaction de condensation, de déshydratation et d'hydrogénation des cétones ou aldehydes est conduite dans un seul réacteur.

La pervaporation se définit comme une combinaison efficace de permeation à travers des membranes, suivie d'évaporation. On décrit habituellement le transport par pervaporation au moyen du modèle de la solution-diffusion. Les étapes sont: la sorption préférentielle des composants à l'interface de la membrane en contact avec l'alimentation liquide, la diffusion différenciée à travers la membrane en raison des gradients de concentration, et, finalement, la désorption sous forme de vapeur, de l'autre côté de la membrane, maintenue à basse pression. Les deux premières étapes correspondent à la permsélectivité de la membrane.

On entend au sens de l'invention par module de pervaporation, une cuve comprenant une ou plusieurs membranes de pervaporation. Les modules de pervaporation comportent généralement des membranes planes, montées sur des supports métalliques, formant des plaques étanches, espacées de façon à avoir le flux de rejet d'un côté et le flux de perméat, comme la vapeur, de l'autre; ces plaques communiquent à travers des collecteurs situés à l'opposé de l'alimentation pour le courant rejeté.

Les membranes de pervaporation sont classées selon la nature de la séparation à étudier. Les membranes hydrophiles sont particulièrement utilisées pour retirer l'eau de solutions contenant aussi des composés organiques. Ces membranes sont typiquement composées de polymères dont les températures de transition vitreuse sont supérieures à la température ambiante. L'alcool polyvinylique est un exemple de matériau de membrane hydrophile. La pervaporation utilise préférentiellement une membrane constituée de polymères présentant une température de transition vitreuse supérieure à la température ambiante. La membrane peut être constituée d'alcool polyvinylique.

La perméabilité des membranes de l'invention peut etre comprise entre 500 à 2000 ml/h.$m^2$, préférentiellement entre 750 et 1500 ml/h.$m^2$, dans le cadre du procédé selon la présente invention.

L'homme du métier est parfaitement au fait de l'emploi de membranes hydrophiles poreuses ou non-poreuses pour déshydrater des composés organiques oxygénés tels que la MIBK. Les brevets US 4910344, CA 2196478, EP 496090, US 4935144, EP 381477 et US 5139677 sont des exemples de réalisations qui concernent la concentration ou le retrait d'eau de solutions organiques aqueuses par pervaporation.

Comme montré dans le schéma de la Figure 1, les réactants tels que les cétones ou aldéhydes, notamment de l'acétone (1) et de l'hydrogène (2) sont amenés dans le réacteur (3). Le courant sortant (4) du réacteur (3) est conduit majoritairement vers des systèmes de purification (8), notamment distillation, et une faible partie de ce courant (5) est détourné vers un module de pervaporation membranaire (6), ou l'eau est partiellement prélevée (11). Le courant partiellement déshydraté (7) est reconduit vers le réacteur (3). Les cétones ou aldéhydes produits et sortants des systèmes

de purification (8) sont récupérés par le moyen (10) ; alors que les cétones ou aldéhydes qui n'ont pas réagit peuvent être ramenés vers le réacteur (3).

On préfère notamment que le taux de reflux de courant (5) amené au module de pervaporation membranaire (6) soit compris entre 20 et 40 %, du courant sortant total (4), sortant du réacteur (3).

La concentration d'eau dans la masse réactionnelle du réacteur est maintenue entre 2 et 4 % en poids. On obtient notamment une réduction de la concentration d'eau dans la masse réactionnelle comprise entre 0,5 et 2 % en poids.

La température dans les modules de pervaporation membranaire peut etre comprise entre 70 et 90°C. La pression absolue dans les modules de pervaporation peut varier entre 5 et 7 kPa. Cette pression correspond à la pression du coté perméat des membranes.

Le procédé de l'invention peut parfaitement comprendre plusieurs modules de pervaporation membranaire, montés en série, de telle manière que la solution soit progressivement desydratée du premier au dernier module. On préfère notamment utiliser trois modules montés en série.

Ainsi par exemple, pour des températures comprises entre 100 et 150°C et des pressions comprises entre 500 et 2000 kPa, une pompe aspire un courant latéral, dont le débit massique est compris entre 20 et 50% du débit massique de sortie du réacteur, alimentant un économiseur et réduisant la température de ce courant, entre 100 et 150°C, à une température d'alimentation des modules entre 70 et 90°C. L'alimentation en poids des modules de pervaporation étant composée d'environ 3 à 5 % d'eau, et 95 à 97% de composants organiques. Le liquide de refroidissement, en opération normale, est le courant rejeté partiellement déshydraté, à la sortie du dernier module de l'ensemble de membranes à une température entre 70 et 90°C et, au démarrage, l'eau de la tour de refroidissement.

Ainsi, le premier module de pervaporation est alimenté à une température entre 70 et 90°C pour débuter la permeation du mélange. Le courant rejeté dans le premier module, à une température inférieure à la température idéale alimente un échangeur de chaleur, utilisant la vapeur comme moyen de chauffage afin d'assurer que l'alimentation du second module se fasse également à une température entre 70 et 90°C.

De la même façon que pour le premier module, la permeation du mélange de perméat se poursuit dans le second module et le courant rejeté, obtenu également à une température inférieure à la température idéale, est dirigé vers le second échangeur de chaleur, utilisant la vapeur comme moyen de chauffage afin d'assurer que l'alimentation du troisième module se fasse également à une température d'approximativement 70 à 90°C.

Le courant final rejeté, déshydraté d'approximativement 1% en poids, et à une température entre 70 et 80°C, alimente l'économiseur et refroidit le courant d'alimentation, comme décrit ci-dessus, étant chauffé jusqu'à une température entre 95 et 145°C avant de retourner au réacteur.

La surface de permeation de chacun des modules peut varier entre 20 et 150 $m^2$, notamment entre 40 et 60 $m^2$. Les membranes peuvent être installées dans une cuve opérant à une pression absolue entre 5 et 7 kPa.

La pression indiquée peut être obtenue par une pompe à vide, installée à la sortie du courant de perméat à l'état de vapeur, requérant un condenseur, alimenté par une solution de saumure, permettant d'obtenir de l'eau à basse concentration d'acétone ou d'aldéhyde. L'installation d'un second condenseur après la pompe à vide est nécessaire pour obtenir une acétone ou un aldéhyde pratiquement pur, qui peut être recyclé dans le procédé.

La présente invention a aussi pour objet un dispositif, tel que ceux classiquement utilisés pour la fabrication de cétones ou d'aldéhyde, comprenant au moins un réacteur (3) et un moyen d'acheminer le courant sortant (4) du réacteur (3) pour récupérer les cétones ou d'aldéhyde produits, et un moyen d'acheminer un courant latéral (5) de sortie du réacteur (3) vers au moins un module de pervaporation membranaire (6), et un moyen d'acheminer le concentré déshydraté résultant (7) dans ledit réacteur (3).

Les quelques exemples à suivre sont simplement à titre d'illustration et ne doivent pas limiter la portée de l'invention.

## PARTIE EXPERIMENTALE

### Exemple 1

Dans un dispositif semblable à celui de la Figure 1 comprenant un module de pervaporation membranaire utilisant une membrane Sulzer PVAP 2256, on alimente la composition suivante :

- Eau : 4,1
- Acétone : 85,2
- MIBK : 10,7

Avec un réacteur fonctionnant à 60°C et en considérant 1% de déshydratation, on obtient un perméat (flux sortant) comprenant 74,5 % en poids d'eau, 25,5 % d'acétone et 0 % en poids de MIBK; avec une perméabilité de 1000

ml/h.m$^2$ de la membrane en fonctionnement. La pression coté perméat de la membrane est de 6 kPa.

Sur la base des résultats de l'essai, l'impact de l'utilisation du module de pervaporation membranaire sur la cinétique de la réaction a été évalué en absence et en présence d'un taux de reflux vers le module de pervaporation ; et les résultats sont les suivants :

**Tableau 1**

| | Exemple comparatif | Exemple selon l'invention |
|---|---|---|
| Taux de reflux (%) | 0 | 25 |
| Production MIBK (tonne/jour) | 100 | 120 |
| Eau sortie du réacteur (%) | 4,2 | 3,4 |

On observe ainsi un rendement accru de la réaction et une forte augmentation de la productivité de MIBK.

**Exemple 2**

Une expérimentation a été effectuée en utilisant 3 modules de pervaporation membranaire, montés en série, de telle manière que la solution soit progressivement desydratée du premier au troisième module.

De l'acétone et de l'hydrogène sont introduits dans le réacteur en vue d'obtenir du MIBK.

À partir du courant de sortie du réacteur (4), une pompe aspire un courant latéral (5) dont le débit massique est égal à 35% du débit massique du courrant de sortie du réacteur (3). Le courant latéral (5) alimente un économiseur, réduisant ainsi la température de ce courant à 80°C. Le liquide de refroidissement, en opération normale, est le courant rejeté (7) de l'ensemble des membranes, à une température de 75°C. Au démarrage, le liquide de refroidissement est l'eau de la tour de refroidissement.

Ainsi, le courant latéral (5) alimente le premier module de pervaporation pour débuter la permeation du mélange de perméat. Le courant rejeté dans le premier module, à une température de 72°C alimente un échangeur de chaleur afin d'assurer que l'alimentation du second module se fasse également à 80°C.

De la même façon que pour le premier module, la permeation du mélange de perméat se poursuit dans le second module et le courant rejeté, obtenu à 74°C, se dirige vers le second échangeur de chaleur, afin d'assurer que l'alimentation du troisième module se fasse également à 80°C.

Le chauffage des courants rejetés dans les échangeurs décrits se fait par l'alimentation en vapeur saturée de chacun des échangeurs de chaleur décrits.

Le courant rejeté final (7), proprement déshydraté, à une température de 75°C, alimente l'économiseur et refroidit le courant d'alimentation du premier module, comme décrit ci-dessus, étant chauffé jusqu'à une température entre 95 et 145°C, avant de retourner au réacteur.

Les trois modules de pervaporation prévus pour l'exécution de l'invention, sont constitués de membranes planes montées sur des supports métalliques, constituant des plaques étanches, espacées de façon à avoir le flux de rejet d'un côté et le flux de perméat, comme la vapeur, de l'autre, communiquant à travers des collecteurs, situés à l'opposé de l'alimentation pour le courant rejeté. Les membranes sont installées dans une cuve opérant à une pression absolue de 6 kPa.

La pression indiquée est obtenue à l'aide d'une pompe à vide, installée à la sortie du courant de perméat à l'état de vapeur, requérant un condenseur, alimenté par une solution de saumure, permettant d'obtenir de l'eau à basse concentration d'acétone. L'installation d'un second condenseur après la pompe à vide est nécessaire pour obtenir une acétone pratiquement pure, qui peut être recyclée dans le procédé.

On observe une forte augmentation de la productivité de MIBK quand une fraction du courant sortant du réacteur est amenée vers les modules de pervaporation.

## Revendications

1. Procédé de production de cétones ou aldehydes obtenus par la condensation suivie de la déshydratation et hydrogénation de cétones ou aldehydes dans un réacteur, **caractérisé en ce que** ledit procédé comprend au moins une étape d'élimination partielle d'eau effectuée par au moins un module de pervaporation membranaire, alimenté tangentiellement et installé latérallement audit réacteur et fonctionnant en boucle, de façon à déshydrater partiellement une fraction sortante du réacteur et à recycler le concentré déshydraté dans ledit réacteur, de sorte que la concentration d'eau dans la masse reactionnelle du réacteur soit maintenue entre 2 et 4 % en poids, et **en ce que**

la cétone est la méthyl isobutyl cétone ou MIBK, obtenu à partir de l'hydrogénation d'acétone, ou le butanal obtenu à partir d'acétaldéhyde.

**2.** Procédé selon la revendication 1, **caractérisé en ce que** le taux de reflux de courant amené au module de pervaporation membranaire est compris entre 20 et 40 %.

**3.** Procédé selon l'une quelconque des revendications 1 ou 2, **caractérisé en ce que** le module de pervaporation comprend au moins une membrane hydrophile.

**4.** Procédé selon l'une quelconque des revendications 1 à 3, **caractérisé en ce que** la membrane du module de pervaporation membranaire comprend au moins un polymère ayant une température de transition vitreuse supérieure à la température ambiante.

**5.** Procédé selon l'une quelconque des revendications 1 à 4, **caractérisé en ce que** la membrane du module de pervaporation membranaire est constituée d'alcool polyvinylique.

**6.** Procédé selon l'une quelconque des revendications 1 à 5, **caractérisé en ce que** la perméabilité de la membrane est comprise entre 500 et 2000 ml/h.$m^2$.

**7.** Procédé selon l'une quelconque des revendications 1 à 6, **caractérisé en ce que** la perméabilité de la membrane est comprise entre 750 et 1500 ml/h.$m^2$.

**8.** Procédé selon l'une quelconque des revendications 1 à 7, **caractérisé en ce que** le procédé comprend plusieurs modules de pervaporation membranaire montées en série.

**9.** Procédé selon l'une quelconque des revendications 1 à 8, **caractérisé en ce que** la température dans le module de pervaporation membranaire est comprise entre 70 et 90ºC.

**10.** Procédé selon l'une quelconque des revendications 1 à 9, **caractérisé en ce que** la pression absolue dans le module de pervaporation membranaire varie entre 5 et 7 kPa.

**11.** Dispositif pour la réalisation du procédé selon l'une quelconque des revendications 1 à 10, comprenant au moins un réacteur (3) et un moyen d'acheminer le courant sortant (4) du réacteur (3) pour récupérer les cétones ou d'aldéhyde produits, et un moyen d'acheminer un courant latéral (5) de sortie du réacteur (3) vers au moins un module de pervaporation membranaire (6), et un moyen d'acheminer le concentré déshydraté résultant (7) dans ledit réacteur (3).

**12.** Dispositif selon la revendication 11 **caractérisé en ce que** ledit dispositif comprend 3 modules de pervaporation membranaire.

## Patentansprüche

**1.** Verfahren zur Herstellung von Ketonen oder Aldehyden, die durch Kondensation erhalten werden, gefolgt von der Dehydratisierung und Hydratisierung von Ketonen oder Aldehyden in einem Reaktor,
**dadurch gekennzeichnet, dass** das Verfahren mindestens einen Schritt der teilweisen Eliminierung von Wasser umfasst, der durch mindestens ein Membranpervaporationsmodul durchgeführt wird, das tangential gespeist wird und lateral von dem Reaktor installiert ist und in einer Schleife arbeitet, um eine Fraktion, die aus dem Reaktor austritt, teilweise zu dehydratisieren und das dehydratisierte Konzentrat in den Reaktor derart zurückzuführen, dass die Konzentration von Wasser in der Reaktionsmasse des Reaktors zwischen 2 und 4 Gew.-% gehalten wird, und dadurch, dass das Keton Methylisobutylketon oder MIBK ist, das aus der Hydratisierung von Aceton erhalten wird, oder Butanal, das aus Acetaldehyd erhalten wird.

**2.** Verfahren nach Anspruch 1,
**dadurch gekennzeichnet, dass** die Rückflussrate des zu dem Membranpervaporationsmodul geführten Stroms zwischen 20 und 40 % beträgt.

**3.** Verfahren nach einem der Ansprüche 1 oder 2,

**dadurch gekennzeichnet, dass** das Pervaporationsmodul mindestens eine hydrophile Membran umfasst.

4. Verfahren nach einem der Ansprüche 1 bis 3,
**dadurch gekennzeichnet, dass** die Membran des Membranpervaporationsmoduls mindestens ein Polymer mit einer höheren Glasübergangstemperatur als Umgebungstemperatur umfasst.

5. Verfahren nach einem der Ansprüche 1 bis 4,
**dadurch gekennzeichnet, dass** die Membran des Membranpervaporationsmoduls aus Polyvinylalkohol besteht.

6. Verfahren nach einem der Ansprüche 1 bis 5,
**dadurch gekennzeichnet, dass** die Permeabilität der Membran zwischen 500 und 2000 $ml/h.m^2$ beträgt.

7. Verfahren nach einem der Ansprüche 1 bis 6,
**dadurch gekennzeichnet, dass** die Permeabilität der Membran zwischen 750 und 1500 $ml/h.m^2$ beträgt.

8. Verfahren nach einem der Ansprüche 1 bis 7,
**dadurch gekennzeichnet, dass** das Verfahren mehrere Membranpervaporationsmodule umfasst, die in Serie angebracht sind.

9. Verfahren nach einem der Ansprüche 1 bis 8,
**dadurch gekennzeichnet, dass** die Temperatur in dem Membranpervaporationsmodul zwischen 70 und 90°C beträgt.

10. Verfahren nach einem der Ansprüche 1 bis 9,
**dadurch gekennzeichnet, dass** der absolute Druck in dem Membranpervaporationsmodul zwischen 5 und 7 kPa variiert.

11. Vorrichtung zur Durchführung des Verfahrens nach einem der Ansprüche 1 bis 10, umfassend mindestens einen Reaktor (3) und ein Mittel zum Leiten des aus dem Reaktor (3) austretenden Stroms (4), um erzeugte Ketone oder Aldehyd zu gewinnen, und ein Mittel zum Leiten eines lateralen Stroms (5) des Ausgangs des Reaktors (3) zu mindestens einem Membranpervaporationsmodul (6) und ein Mittel zum Leiten des erhaltenen dehydratisierten Konzentrats (7) in den Reaktor (3).

12. Vorrichtung nach Anspruch 11,
**dadurch gekennzeichnet, dass** die Vorrichtung 3 Membranpervaporationsmodule umfasst.

## Claims

1. Process for producing ketones or aldehydes obtained by condensation followed by dehydration and hydrogenation of ketones or aldehydes in a reactor, **characterized in that** said process comprises at least one step of partial elimination of water carried out by at least one membrane pervaporation module, fed tangentially and installed laterally to said reactor and operating in a loop, so as to partially dehydrate a fraction exiting the reactor and to recycle the dehydrated concentrate to said reactor, such that the concentration of water in the reaction mass of the reactor is maintained between 2% and 4% by weight and **in that** the ketone is methyl isobutyl ketone or MIBK, obtained from the hydrogenation of acetone, or butanal obtained from acetaldehyde.

2. Process according to Claim 1, **characterized in that** the degree of reflux of stream brought to the membrane pervaporation module is between 20% and 40%.

3. Process according to either one of Claims 1 and 2, **characterized in that** the pervaporation module comprises at least one hydrophilic membrane.

4. Process according to any one of Claims 1 to 3, **characterized in that** the membrane of the membrane pervaporation module comprises at least one polymer having a glass transition temperature above ambient temperature.

5. Process according to any one of Claims 1 to 4, **characterized in that** the membrane of the membrane pervaporation module consists of polyvinyl alcohol.

6. Process according to any one of Claims 1 to 15, **characterized in that** the permeability of the membrane is between 500 and 2000 ml/h.m$^2$.

7. Process according to any one of Claims 1 to 6, **characterized in that** the permeability of the membrane is between 750 and 1500 ml/h.m$^2$.

8. Process according to any one of Claims 1 to 7, **characterized in that** the process comprises several membrane pervaporation modules mounted in series.

9. Process according to any one of Claims 1 to 8, **characterized in that** the temperature in the membrane pervaporation module is between 70 and 90°C.

10. Process according to any one of Claims 1 to 9, **characterized in that** the absolute pressure in the membrane pervaporation module ranges between 5 and 7 kPa.

11. Device for carrying out the process according to any one of Claims 1 to 10, comprising at least one reactor (3) and a means for conveying the stream (4) exiting the reactor (3) in order to recover the ketones or aldehydes produced, and a means for conveying a side stream (5) exiting the reactor (3) to at least one membrane pervaporation module (6), and a means for conveying the resulting dehydrated concentrate (7) to said reactor (3).

12. Device according to Claim 11, **characterized in that** said device comprises 3 membrane pervaporation modules.

FIGURE 1

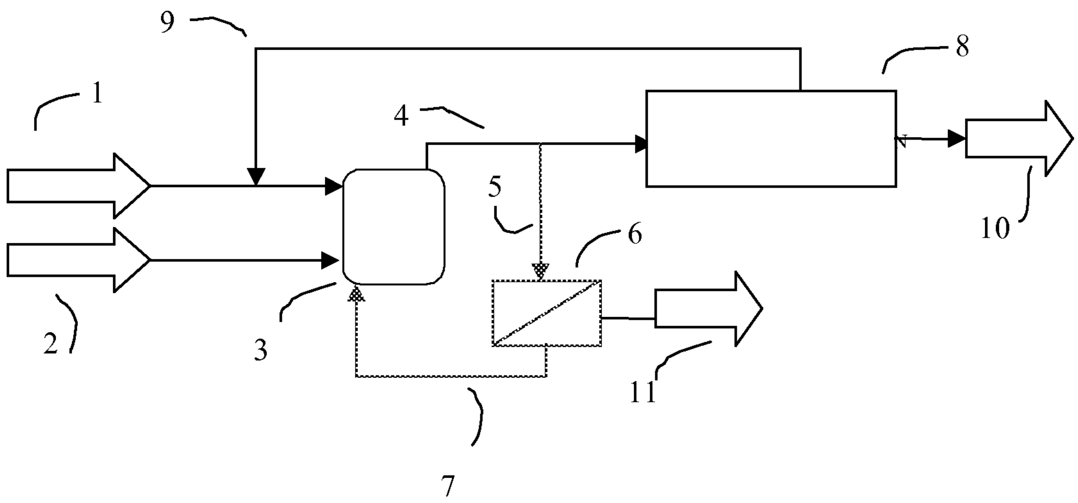

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4910344 A
- CA 2196478
- EP 496090 A
- US 4935144 A
- EP 381477 A
- US 5139677 A


### Littérature non-brevet citée dans la description


- Intégration of pervaporation for the removal of water in the production process of methylisobutylketone. *Journal of Membrane Science,* 1996, vol. 111, 135-141